# EUROPEAN PATENT APPLICATION

(11) **EP 4 553 104 A1**
(43) Date of publication of application: **14.05.2025**
(21) Application number: 24211932.9
(22) Date of filing: 08.11.2024
(51) Int. Cl.: C08J 3/075, A61K 47/42, A61L 27/24, B33Y 70/00, C08J 3/09, C08J 5/18, C08J 9/28, C08L 89/06

(54) **METHOD FOR PREPARING HOMOGENEOUS COLLAGEN SUSPENSION, USE OF HOMOGENEOUS COLLAGEN SUSPENSION, METHOD FOR PREPARING COLLAGEN-BASED HYDROGEL MATERIALS, METHOD FOR PREPARING COLLAGEN FILM AND/OR LAYER AND/OR SUBSTRATE FOR CULTURE, METHOD FOR PREPARING COLLAGEN PRINT-OUTS, AND METHODS FOR PREPARING POROUS COLLAGEN SHAPES**

(30) Priority: 08.11.2023 PL 44667523
(71) Applicant: Akademia Gorniczo-Hutnicza im. Stanislawa Staszica w Krakowie, 30-059 Krakow (PL)
(72) Inventor: Benko, Aleksandra, 31-637 Kraków (PL); Dziadek, Michal, 32-083 Balice (PL); Wilk, Sebastian, 31-049 Kraków (PL); Pietryga, Krzysztof, 30-052 Kraków (PL)
(74) Representative: Augustyniak, Magdalena Anna

(57) **Abstract**

An object of the invention is a method for obtaining a homogeneous collagen suspension, characterized in that it comprises the following steps
**I.** to collagen, with mixing, preferably on a vortex, a solution composed of a mild organic solvent and a buffer, with the addition of acids is added wherein:
- as the collagen preferably type I collagen is used, preferably it is the collagen derived from bovine tendons,
- the concentration of the collagen with respect to the solvent is in the range of 0.1 to 15% w/v,
- the organic solvent is a solvent exhibiting low surface tension relative to the collagen, preferably the solvent is a solvent for which the contact angle value for collagen is below 20°, most preferably the organic solvent is dimethyl sulfoxide (DMSO),
- the buffer is a buffer containing salts naturally occurring in a living organism, preferably the buffer is a phosphate buffer (PBS),
- the solvent and the buffer can be mixed together in various proportions, preferably the solvent and the buffer are mixed in a ratio of 5 parts of the solvent to 1 part of the buffer, by volume, most preferably 5 parts DMSO to 1 part PBS, by volume,
- the acids are HCl and HF, wherein the concentration of each of the acid in the solution is not higher than 0.1 M,

**II.** subsequently, the solution obtained in step **I.** is mixed at a temperature of 2 - 8°C, preferably 4 - 6°C, for a period of 3 to 7 days, preferably for a period of 3 days,
**III.** subsequently, the solution obtained in step **II.,** while mixing, preferably on a vortex, is gradually acidified using an acid solution, to a pH in the range of 2 to 4, preferably to pH 2.5, preferably the acid solution is 1 M HCl.
**IV.** subsequently, the solution obtained in step **III.** is mixed at a temperature of 2 - 8°C, preferably 4 - 6°C, for a time necessary to further reduce the number of agglomerates in the solution, preferably, for a period of 3 days,
**V.** subsequently, the suspension obtained in step **IV.** is homogenized to obtain a homogeneous collagen suspension, preferably first the suspension being homogenized mechanically, and then the mixture being homogenized manually.

An object of the invention is also the use of a homogeneous collagen suspension obtained by the method according to the invention.

An object of the invention is also a method for preparing collagen-based hydrogel materials, a method for preparing a collagen film and/or layer and/or substrate for culture, a method for preparing collagen print-outs, and methods for preparing porous collagen shapes.

## Description

### Field of invention

The invention is directed to a method for preparing a homogeneous collagen suspension, use of a homogeneous collagen suspension, a method for preparing collagen-based hydrogel materials, a method for preparing a collagen film and/or layer and/or substrate for culture, a method for preparing collagen bioprint-outs, and methods for preparing porous collagen shapes.

The method for preparing a homogeneous collagen suspension, preferably of type I, comprises preparing homogeneous suspensions based on the principles of green chemistry, and further enables obtaining forms of collagen with desired shapes, morphologies and sizes, and mechanical handiness (films, layers, injectable materials, shapes produced by 3D printing techniques, by mould casting or direct printing using extrusion techniques).

The subject items can primarily find application in broadly understood biomaterial engineering and biology. They can be used: as substrates in cell and tissue culturing, in the development of hydrogel dressing materials, in the development of injectable biomaterials. In addition, such collagen products can also find application as sorbents, bioseparation materials, membranes for filtration/biofiltration, selective separation, adsorption, catalysts and carriers of various types of active substances (e.g., drugs, fertilizers).

### Background of invention

Processing various types of biopolymers and polymers into desired forms and shapes has been the subject of numerous research efforts and patent literature. The most popular morphologies include 3D structures, fibres (including nanofibres), films and membranes. Polymers, and recently also biopolymers, are commonly used in many fields of science and industry. Since biopolymers are most often by-products of various industrial processes, including food production, their use for various applications is consistent with the idea of closed-loop economy and the so-called green chemistry. Another advantage of biopolymers is their biodegradability.

One of the most popular and essential applications of biopolymers are in problems at the interface of biology, biotechnology and materials engineering, including techniques of cell culturing and tissue engineering. The main reason for this is the fact that biopolymers provide optimal biomimicry, i.e., they are able to reproduce the natural biological environment in which cells function in a living organism. Current research trends suggest performing cell culturing on specially prepared 3D substrates, which increases results' translatability of research conducted in vitro to tests in vivo. In turn, better results' translatability means viable reduction in the costs of developing new drugs and therapeutic strategies.

Of the many biopolymers known in technology, the most common are proteins, of which collagen is most commonly used. Collagen is the predominant building component of animals, available in significant quantities in by-products of food and clothing manufacturing: in tendons, joints, bones, internal organs and skin. Since collagen is a byproduct of many production processes, it fits perfectly into the trend of a closed-loop economy. On the other hand, the fact that it is present in a large number of animal tissues is a factor determining its high biomimicry - it reflects the natural environment of cells, because it is the main component of the extracellular matrix making up the tissues in which these cells live. In this respect, of particular importance is collagen type I, which has a natural ability to form fibrils and fibres, has good mechanical properties and is the most prevalent type of collagen, constituting the main building component of many tissues.

Processing biopolymers (after prior isolation from tissues) into desired shapes is not an easy process and it involves careful optimization of the following steps:
1. selection of solvent,
2. selection of homogenization method (suspension unification),
3. selection of technique for imparting appropriate shape (most often it is casting),
4. selection of appropriate neutralization/cross-linking strategy, which will allow obtaining a stable material that does not dissolve in water and retains its shape and form for the desired period of time.

In the case of collagen, processing is difficult due to the relatively low solubility with preservation of the native structure (i.e., dissolution in such a way that collagen does not denature and its chain does not fragment, for example to form gelatine). Denaturation is undesirable because it reduces the similarity of the obtained material to structures naturally occurring in a living organism, and reduces long-term stability, which may require the use of aggressive cross-linkers. This in turn makes the process less "green". In the case of collagen, the preservation of native structure makes the material, after processing and without the use of an additional cross-linker, not soluble in water. Hence, from a scientific and industrial point of view, there is a high demand for the development of a collagen dissolution methodology to obtain homogeneous suspensions, from which chemically and physically stable materials of the desired shapes and sizes can then be obtained, without the need for additional chemical or thermal cross-linking, the use of which lowers the biomimicry of the materials.

If collagen denatures to gelatine, its dissolution becomes easy, but in this way it loses its biomimicry, and processing to the desired shape usually requires chemical modification. Processing gelatine to desired shapes has been the topic of many scientific publications, and is known in the art and disclosed in numerous patent applications. An example of chemical modification is the state-of-the-art methacrylated gelatine (GelMA, e.g., Stemorgan Inc.). On the other hand, as to the known cross-linkers, one can mention photo-cross-linkers, such as Igracure 2959 (e.g., Cellink protocol: https://www.cellink.com/wp-content/uploads/2018/04/GelMACastingProtocolver 1 .pdf), or chemical cross-linkers, such as 1-ethyl-3-(3-dimethylaminopropyl)carbodiimide hydrochloride (EDC, used for example in publication N640CHE2013A), glutaraldehyde (as in patent document EP3921372B1), and other cross-linkers commonly used and known in the art, including in particular those in which cross-linking is initiated by activating the functional groups of gelatine (and collagen), in particular via an amide bond, such as N,N'-dicyclohexylcarbodiimide (DCC), carbodiimide, or thionyl chloride.

Cell culturing substrates, which comprise collagen (especially type IV) as well as other biopolymers, are also known in the art. Examples include Matrigel^{®} and Geltrex^{®}. In both cases, a low-concentration material is added to the culture medium and a small amount of fibres settles at the bottom of the culture wells. There is no possibility of processing such substrates into more stable and easy to handle structures, including 3D structures.

In the art, there is no solution that would combine the advantages of using substrates based on pure collagen with the possibility of shaping available for gelatine materials, while at the same time relying on the assumptions of green chemistry, especially in terms of the solvent used.

Among the methods of collagen dissolution known in the art, two basic types of solvents can be distinguished:
1. aggressive organic solvents, among which prevail toxic halogens, primarily hexafluoroisopropanol, and
2. acidic solvents, wherein organic acids (especially acetic acid) or inorganic acids (usually HCl) are used to reduce the pH value to about 2.

The first strategy does not fit into the framework of green chemistry and often causes denaturation of the material. The second, with an appropriately selected methodology, allows collagen processing in a "green" way, without its denaturation. In such a favourable case, the dissolution reaction is completely reversible, and the precipitation/gelation of collagen takes place by neutralizing the biopolymer solution with a solution of any base, e.g., sodium hydroxide (US20040028738A1).

Collagen dissolution and gelation based on "green" solvents is a topic addressed in many applications.

US20040028738A1 describes a method for producing a porous collagen matrix obtained by processing fat- and hair-free pigskin. The skin is fragmented, digested with pepsin and dissolved in acids (0.5M acetic acid is provided as the preferred acid). The solution is then neutralized to a pH in the range of 5 - 9, e.g., by dialysis into a buffer (preferably a phosphate buffer). The use of a buffer with the addition of appropriate salts (e.g., NaCl) can also be used to obtain the desired pore size in the lyophilization process, which is also a topic of the discussed publication. The solution thus prepared is then incubated at a temperature between about 30 and about 45°C (preferably 37°C) for a minimum of 0.5 h, preferably 24 h, to form a collagen gel (fibrillation), without removing the liquid. The gel is then frozen and freeze-dried, wherein the freezing rate and target temperature are selected to obtain the desired porosity (the exact effect of the freezing rate and target temperature on the size and distribution of pores is discussed in the publication). Preferably, a cooling rate greater than 10°C and a target temperature of -80°C give a fibrous collagen structure with a uniform distribution of pores in the range of 30 µm. After freeze-drying, the porous collagen is subsequently soaked in a liquid organic compound that wets well the collagen (low surface tension between collagen and liquid), preferably ethanol, the patent also mentions dimethyl sulfoxide (DMSO). The use of this last step is intended to prevent matrix shrinkage. In the discussed publication, only acids are used to obtain the collagen suspension, and there are no additional steps of homogenization of the solutions. Little emphasis is given to obtaining a homogeneous suspension, and the preparation time of the suspension is not considered. In order to preserve the desired morphology, production of the material requires the use of final soaking of shapes in an organic solvent - thus additional post-production processing is necessary. This publication does not address the issue of storage of the materials, their long-term chemical and mechanical stability, and possible cytotoxicity of residual solvent when using the finally obtained material as a biomaterial. Additionally, this publication proposes only one form and method for production (lyophilization to obtain porous substrates), which significantly limits the potential applications.

US5541234A discloses a method for producing highly porous shapes using biopolymers and synthetic polymers, wherein a common feature is a high content of hydroxyl groups - thus the ability to form hydrogels. The proposed materials include: alginates, gums, carrageenan, starch, dextrins, agar, gelatines, casein, collagen, polyvinyl alcohol, polyethyleneimine, acrylate polymers, polyalkylene amides, polyacrylamides, polyalkylene imides, and any copolymers of all of the foregoing. Polymers or their mixtures are gelled at a concentration of 0.02% to 15% in an undisclosed solvent; only water is provided in the embodiments. Where necessary, hydration takes place in the presence of an additional gelling agent known in the art; gelation by rapid cooling is also possible. Here, it should be noted that some of the materials listed, including collagen, are not soluble in water, and the provided method in most cases only assumes hydration of the material (partial hydration), and not its dissolution. In the case of agar, carrageenan, gelatine and casein, the disclosed technology assumes dissolution in water at 80°C. There is no corresponding information for collagen, although in its case heating to 80°C will lead to denaturation. Additionally, the disclosed method does not provide any information on the hydration methodology and the importance of preparing a homogeneous suspension for the final properties of the polymer. The hydrated/gelled polymer/copolymer can then be formed into any shape, the publication providing casting into moulds, casting films, shaping fibres or particles. The method further assumes replacing the gelling agent with a cross-linking solvent.

The cross-linking solvent should have a surface tension equal to or lower than that of the gelling liquid (preferably below 75 dynes/cm), allowing diffusion and liquid exchange to occur. DMSO is mentioned as one cross-linking solvent. In a preferred embodiment, the gelling liquid is completely replaced by the cross-linking solvent via diffusion processes (with the possible use of an intermediate step with additional liquid and/or by exchange at a concentration gradient - increasing concentration of the cross-linking solvent to minimize pore collapse). Alternatively, the liquid exchange can be performed by other methods known in the art: freeze-drying or supercritical fluid extraction. Then, the material is cross-linked using any cross-linker known in the art that is able to bind -OOH, NH₂ or -OSO₃ groups. Diamines and polyamines, preferably diisocyanates, are mentioned as preferred cross-linkers. It should be noted that the proposed cross-linkers are generally toxic. The duration and temperature of the cross-linking are selected experimentally, and after the process is completed, the residue of the cross-linker dissolved in the organic solvent is removed by methods known in the art, including in particular drying at 20°C at reduced pressure. The obtained material is characterized by open porosity, density of less than about 1.0 g/cm³, surface area ≥ 30 m² and compressive strength equal to or less than about 75% of the yield strength at 300 psi. This publication does not provide the methodology of collagen processing. The publication also does not address the issue of materials' storage, their long-term chemical and mechanical stability, and possible cytotoxicity of the cross-linker and solvent residues when using the finally obtained material as a biomaterial.

CN112920427A describes a method for preparing a hydrogel, comprising the following steps: the precursor solution is subjected to directed freezing (controlled cooling rate, to a controlled temperature) and drying to obtain a hydrogel precursor, which is then immersed in water to obtain a hydrogel. Improvement of mechanical properties is obtained by directed crystallization in the freezing step, which can be achieved by methods known in the art: adding crystallization seeds to the precursor solution, applying external stress during freezing, or modifying the hydrogel precursor itself. The hydrogel obtained in this way has an ordered structure, nanocrystalline domains, and directionally arranged pores. The hydrogel is given shape by casting into moulds of the desired shape, also those obtained by 3D printing techniques (and other methods known in the art), and then lyophilization. The hydrogel used may be a synthetic polymer (including PEG, PVA, polyacrylamides) or a biopolymer (including collagen, chitosan), as well as their copolymers. Water or organic liquids, including DMSO, are suggested as solvents. The concentrations used are in the range of 0.5 - 50%; preferably, an inorganic additive is added to the material. However, this publication does not include a collagen processing methodology, nor are there any additional solution homogenization steps. Little emphasis is also given to obtaining a homogeneous suspension, and the preparation time of the suspension is not considered. The publication does not address the issue of materials' storage, their long-term chemical and mechanical stability.

WO2018204315A1 describes a method for producing an implant making use of a hydrogel, which may be a hydrogel containing collagen and/or a non-biodegradable polymer (e.g. polyvinyl alcohol, PVA). The publication discusses the use of prefabricated, porous shapes (sponges), made of, e.g., gelatine, collagen, polylactide, polyglycolide, chitosan, alginate, PVA or polyethylene glycol (PEG). The sponges are hydrated in water and then the liquid is replaced with an organic solvent, preferably DMSO (similarly to US5541234A and US20040028738A1). In the final step, the resulting shapes are freeze-dried. In another embodiment, the dissolved polymer (including hydrogel) is extruded in a controlled manner into moulds. The publication mainly discusses various strategies for obtaining materials with a hierarchical microstructure, in which solid and porous areas are predetermined. However, this publication does not discuss the processes of dissolving and cross-linking collagen. There are also no steps of additional solution homogenization. Little emphasis is given to obtaining a homogeneous suspension, and the preparation time of the suspension is not considered.

US20220175534A1 describes production of collagen microspheres and collagen bioink, obtained using a collagen (type I, II, III or IV) suspension. The collagen suspension is fibrillated by increasing the temperature or using an ionic liquid. The process of preparing a suspension of the desired concentration assumes the use of a precursor in the form of commercially available collagen suspensions. In an embodiment, porcine collagen type I is used, probably suspended in 0.1 M HCl (Porcogen, 10 mg/ml Sunmax Biotechnology, Taiwan). The first step of the preparation is precipitation of collagen from the solution, which is performed by adding 1.04 M NaCl solution and centrifuging to form a pellet, at a speed of 3500 rpm, for 15 minutes. The pellet is then dissolved for 12 hours in 0.5 M acetic acid at a concentration of 1% by weight (acetic acid can also be replaced with ethanol, acetone, acetonitrile or a mixture of all these 4 substances) and dialyzed in a 35 mM solution of EDTA (which acted as a chelating agent for divalent ions; (ethylene glycol)-O-O'-bis(2-aminoethyl)-N,N,N',N'-tetraacetic acid (EGTA) can also be used), adjusted to pH 7.4 using 10 M NaOH. From the 1% collagen suspension thus obtained, microparticles are obtained (by emulsification in oil and sorbitan monostearate/monooleate), which can be conjugated with bioactive substances. The same suspension can also be used as a bioink. In an embodiment, collagen is printed into 15 ml of double-distilled water at 24°C, placed inside a polystyrene Petri dish 150 mm in diameter. Printing is performed with the following parameters: layer thickness 0.2 mm, printing speed 3 mm/s, efflux pressure - 10 kPa. After printing, the materials are left for 5 min to fibrillate and transferred to 70% ethanol for storage. Alternatively, printing is performed using the FRESH technique, into a suspension of agarose particles. The materials can also be cross-linked by adding the cross-linker directly to water/agarose or by washing after printing with a cross-linker solution. The proposed cross-linkers are 1-ethyl-3-(3-dimethylaminopropyl)-1-carbodiimide hydrochloride (EDC) + N-hydroxysuccinimide (NHS) or genipin. It should be noted that the cross-linkers proposed in the publication are generally toxic. The proposed solution assumes printing with a very low collagen concentration, using organic solvents that do not fulfil the principles of green chemistry. This publication does not discuss collagen dissolution, and there are no steps of additional homogenization of the solutions. Little emphasis is given to obtaining a homogeneous suspension, and the preparation time of the suspension is not considered.

AU2001249079B2 discloses a method for producing cohesive coatings by using a dissolved protein suspension that is applied to surfaces, dried and pressed. The suspension contains one or more biocompatible protein materials combined with one or more biocompatible solvents, with or without the addition of one or more pharmaceutically active substances. The publication assumes the use of all methods for producing coatings that are known in the art. The protein used may be synthetic or natural (including collagen, elastin, albumin, keratin, fibronectin, silk and its derivatives, actin, myosin, fibrinogen, thrombin, atroponin, antithrombin II) and the solvent may be water, DMSO, organic acids, alcohols, oils, glycols, etc. The publication does not discuss the process of dissolving and cross-linking collagen. There are also no steps of additional solution homogenization. Little emphasis is given to obtaining a homogeneous suspension, and the preparation time of the suspension is not considered.

US3393080A discloses a composition comprising a dispersion of microcrystalline collagen at a concentration of at least 10% by weight and tropocollagen at a concentration of up to 0.75% by weight. The size of microcrystallites is up to about one micron (in any dimension). Publication discloses the advantageous use of 0.01 M hydrochloric acid (with minimum concentration of 0.0025 M, which is selected according to the concentration of collagen in the solution) for processing cowhides to obtain microcrystalline collagen. It has also been shown that too high a concentration of HCl (from 0.025 M up) causes undesirable degradation of collagen to tropocollagen. In an embodiment, dissolution leading to a 7.5% by weight collagen solution is done using 0.03 M HCl. The collagen solution in HCl is then diluted with an organic solvent miscible with water (e.g., isopropanol) to impart antibacterial properties to the material. The amount of organic solvent should not exceed 65% by weight (above this value collagen precipitates), preferably it should be in the range of 25-35%. If DMSO is added to the solution, the amount of the second organic solvent can be increased to 75%. Suspensions prepared in this way are used to produce thin layers, dried using a lyophilizer. The invention therefore includes the use of DMSO and HCl, where HCl is used at a concentration similar to that proposed in our invention (in the publication, between 0.0025 and 0.025 M, preferably 0.01, in the technical solution according to the invention, between 0.0025 and 0.01 M, preferably 0.005). In the case of publication US3393080A the purpose of using DMSO comes down to enabling the use of higher concentrations of organic solvents. In our case, DMSO is the only organic solvent used, which increases the potential biocompatibility of the material and the so-called "greenness" of the process. However, the publication does not discuss methods of dissolving and cross-linking collagen. There are also no steps of additional homogenization of solutions. Little emphasis is given to obtaining a homogeneous suspension, and the preparation time of the suspension is not considered. Collagen obtained is used for the production of thin layers only, other forms of materials not being discussed. The document does not discuss the issue of materials' storage, their long-term chemical and mechanical stability, and possible cytotoxicity of cross-linker and solvent residues when using the finally obtained material as a biomaterial.

US8198408B2 describes preparation of a porous collagen matrix directly from connective tissue, devoid of fat, hair and hair follicles, by methods known in the art. Examples of origin stocks include animal skin, tendons, cartilage, etc. The methodology describes the use of a PBS solution for initial washing of tissue from contaminants, and a mixture of an organic solvent (including DMSO) and water for hydration and swelling of tissues, which facilitates subsequent penetration of acids into the material. In a further step, the tissue is soaked (swollen) in an organic or inorganic acid, or a mixture thereof, to a stage of increasing the initial thickness by at least 50%. In an embodiment, acetic acid is mentioned with a concentration in the range of 0.1 to 6 M and pH 2-6. Acceleration of the swelling step can be achieved by irrigation with a pressurized acid stream or by using an ultrasonic basin. Then, the soaked tissue is rinsed with a solution that may contain detergent and/or chelating agents and/or proteolytic enzymes, in order to get rid of non-collagen contaminants. The swollen matrix thus prepared can then be slowly frozen (freezing rate is not defined), to a temperature of -20°C and lyophilized or stored in a PBS solution at 4°C (permissible storage time is not defined). Optionally, the material can be subjected to additional cross-linking by methods known in the art. Additionally, the object of publication US8198408B2 is only the processing of the hydrated matrix, and not the dissolution of collagen and subsequent processing into desired shapes, which is the object of our invention.

Publication US3758660A describes a method for producing three-dimensional collagen shapes obtained from a highly concentrated suspension of water-insoluble collagen (15 to 90%). An embodiment mentions collagen type I derived from cowhide. The shapes include, for example, sheaths for cold cuts (sausages, hot dogs, etc.), threads, and cylinder-shape-rolled edible films. The material is obtained by treating native collagen with an acid at a pH in the range of 1.6 - 2.6, and then mechanically fragmenting it until at least 1% of the collagen particles in the suspension (preferably 25%, and most preferably above 85%) have a size below 1 µm. The collagen so obtained is referred to as a partially ionized collagen salt and is characterized by the fact that the content of bound acid is below stoichiometric (not all amino functional groups of collagen are bound with acid). The invention assumes the use of any organic and inorganic acids (and their salts), HCl and citric acid being mentioned as preferred. An appropriate amount of collagen is combined with acid and mechanically mixed using methods known in the art. The minimum water content in the mixture is 5% by weight. To improve mechanical properties, it is permissible use plasticizers, and DMSO is mentioned as one of the examples. The mixture is then heated and shaped using methods known in the art: extrusion, transfer moulding, hot pressing. Process temperatures range between 50 and 125°C. The publication assumes the use of HCl for swelling of the material, partial hydrolysis and formation of collagen salts, DMSO is used as a plasticizer and there is no mention of its effect on collagen dissolution. The object of publication US3758660A is only the processing of hydrated matrix, with subsequent processing including temperatures leading to collagen denaturation.

IN640CHE2013A (entitled: A method for preparing three dimensional collagen fibre mat using benign solvent and products thereof) describes a method for obtaining fibrous collagen mats. Collagen type I, II, III, V or XI (also mixtures thereof) is dissolved in a mixture of 75-99% acetic acid and 1-25% DMSO. In an embodiment, the collagen concentration is 12% and the suspension is obtained by continuous mixing (the mixing method and time are not disclosed). A polymer (for example, polycaprolactone (PCL) or polyhydroxybutyrate (PHB)), dissolved in a suitable solvent (not given), is added to the mixture. The material is then electrospun (a process known in the art) to obtain fibres with a diameter of 50 nm to 2 µm, and the resulting mats are cross-linked by immersion in a 170 mM solution of EDC. Next, they are washed in a PBS solution with the addition of antibiotics. The mats obtained are cytocompatible and stable for up to 9 months (storage conditions are not provided). The publication assumes dissolving collagen in a mixture of acetic acid and DMSO. Little emphasis is given to obtaining a homogeneous suspension, and the preparation time of the suspension is not considered. The obtained collagen is used only for the production of fibres, which in fact are not 100% composed of collagen, but of collagen combined with another synthetic or natural polymer (PCL or PHB). The publication contains information on the stability of the material over time, but there is no information on the method of storage.

US10046087B2 describes a method for producing a polymeric material based on functionalized collagen and/or functionalized collagen derivatives, wherein the functionalization comprises attaching unsaturated ethylene derivatives, including in particular vinyl derivatives, particularly 4-vinylbenzyl chloride (4VBC). The functionalization also comprises modifying the collagen with glycidyl methacrylate. The starting collagen is collagen type I, II, III or IV, and mixtures thereof. The functionalization can take place by any methods known in the art, preferably by electrophilic substitution. The first reaction step is the dissolution of collagen in any solvent known in the art, preferably an aqueous acid solution. In an embodiment, HCl is provided at a concentration of up to 0.005 M (preferably up to 0.001 mM). The collagen is dissolved at a concentration of 0.2 to 1% by weight. Preferably, the dissolution takes place at a temperature of 25°C, for 24 h. Subsequently, the material can be neutralized, preferably by adding NaOH, to obtain a pH in the range of 7.2 - 7.5. It is also possible to add a surfactant known in the art (e.g. Tween^{®}, Pluronic^{®}), in an amount of 0.1 to 2% by weight, which is intended to increase the solubility of the monomers of unsaturated ethylene derivatives. The monomer is then added to the mixture, in an amount relative to lysine in collagen, in a ratio of 5:1 to 150:1 (monomer:lysine). The reaction can be additionally catalysed by any catalyst known in the art, for example triethylamine, in a ratio relative to the monomer of 1:5 to 2:1 (catalyst:monomer). The reaction lasts for up to 24 hours and can be stopped by any known technique, e.g., by dialysis, and the collagen itself can be recovered by any known technique, e.g., by centrifugation. The final degree of functionalization is in the range of 5 mol% to 100 mol%.

Collagen cross-linking is carried out by dissolving 0.5 to 1.5% of collagen in any aqueous solvent, preferably a PBS solution or 0.001 M HCl (pH unknown). The collagen is mixed at 4°C, for 24 to 60 hours, after which the cross-linker is added. The material is cross-linked by adding unsaturated ethylene derivatives, photo-cross-linking using a photo-cross-linker or radiation cross-linking. After cross-linking, the material is washed by incubation in water or a PBS solution for 24 to 72 hours. Preferably, the final product has a preserved triple helix structure and the degradation time in water is up to 60 days.

The publication suggests processing collagen into fibres, films, filaments or substrates. As an example of processing, the so-called wet spinning is provided, in which collagen is dissolved at a concentration of 5 to 16% in any solvent, preferably water-based (20×, 10×, and 5× PBS are suggested, mixed with ethanol in a ratio of 1:1 to 5:1 (PBS:ethanol). Duration of the dissolution is between 24 and 72 h, at any temperature, preferably at 25°C. Spinning takes place in a coagulation bath; as examples of liquids ethanol, acetone, methanol and their mixtures are provided.

The publication does not contain practical examples of processing collagen into forms other than fibres.

The publication discloses functionalized collagen, which is stable due to the use of chemical modification and an additional, potentially toxic cross-linker. Collagen dissolution takes place primarily in an acid, PBS and ethanol also being proposed, however no addition of other organic substances is suggested. There is no analysis of the degree of dispersion of the biopolymer in the liquid and its effect on the final product properties. The possibilities of processing into forms other than fibres are not demonstrated.

CN110713727A discloses a method for producing a collagen hydrogel. The method comprises soaking collagen in an acetic acid solution, and subsequent adjustment of the pH of the mixture using HCl, and aging at a low temperature.

CN103333508A discloses an injectable collagen hydrogel produced using, among others, collagen as a raw material, and a cross-linking agent. The method for preparing same comprises using HCl as a pH-regulating agent.

The constitution of the tested composition provided in the present patent has been partially disclosed in a conference publication of the Biomaterials in Medicine and Veterinary Medicine Conference, which took place 13-16.10.2022 in Rytro. In a summary (http://biomat.krakow.pl/files/konferencje/rytro/rytro_2022_conference_proceedings.pdf , p. 114) in the section "Materials and methods" a collagen solution was described, which was prepared from 1% (w/v) collagen in DMSO/PBS (5:1) with the addition of 1 M HCl (0.1% v/v DMSO/PBS) and 5% HF (0.1% v/v DMSO/PBS). However, the publication neither disclose nor suggests all the steps of the method for obtaining a homogeneous collagen suspension according to the present invention and their effects.

### Objective of invention

The objective of the invention is to propose a new method for processing collagen, preferably of type I, into a homogeneous suspension, based on the principles of green chemistry, which enables obtaining homogeneous collagen materials of the desired shape, size and morphology - films, layers, 3D substrates, print-outs, injectable materials, in a repeatable manner.

### Object of invention

An object of the invention is disclosed in the enclosed set of patent claims and is described in more detail below.

An object of the invention is a method for obtaining a homogeneous collagen suspension, characterized in that it comprises the following steps
**I.** to collagen, with mixing, preferably on a vortex, a solution composed of a mild organic solvent and a buffer, with the addition of acids, is added, wherein:
   - the concentration of the collagen with respect to the solvent is in the range of 0.1 to 15% w/v,
   - the organic solvent is a solvent exhibiting low surface tension relative to collagen,
   - the buffer is a buffer containing salts naturally occurring in a living organism,
   - the solvent and the buffer can be mixed together in various proportions,
   - the concentration of each of the acid in the solution is not higher than 0.1 M,
**II**. subsequently, the solution obtained in step **I.** is mixed at a temperature of 2 - 8°C, preferably 4 - 6°C, for a period of 3 to 7 days, preferably for a period of 3 days,
**III**. subsequently, the solution obtained in step **II**., while mixing, preferably on a vortex, is gradually acidified using an acid solution, to a pH in the range of 2 to 4, preferably to pH 2.5,
**IV.** subsequently, the solution obtained in step **III.** is mixed at a temperature of 2 - 8°C, preferably 4 - 6°C, for a time necessary to further reduce the number of agglomerates in the solution, preferably, for a period of 3 days.
**V.** subsequently, the suspension obtained in step **IV.** is homogenized to obtain a homogeneous collagen suspension, preferably first the suspension being homogenized mechanically, and then the mixture being homogenized manually.

Preferably, in step **I.** the collagen used is type I collagen, preferably the collagen derived from bovine tendons.

Preferably, in step **I.** the concentration of the collagen with respect to the solvent is in the range from 0.1 to 10% w/v, preferably from 0.1 to 5% w/v, equally preferably in the range from 5 to 10% w/v, more preferably 7% w/v, or preferably 5 - 15% w/v.

Preferably, in step **I.** the solvent is a solvent for which the contact angle value for collagen is below 20°.

Preferably, in step **I.** the organic solvent is dimethyl sulfoxide (DMSO).

Preferably, in step **I.** the buffer is a phosphate buffer (PBS).

Preferably, in step **I.** the solvent and the buffer are mixed in a ratio of 5 parts DMSO to 1 part PBS, by volume.

Preferably, in step **I.** HCl and HF acids are added, wherein the minimum concentration of HCl is 0.001 M and the minimum concentration of HF is 0.001 M, more preferably the concentration of HCl is 0.005 M and the concentration of HF is 0.012 M.

Preferably, in step **III**. the acid solution is 1 M HCl acid.

Preferably, in step **V.** the collagen is admixed with glycerol in an amount of 0.1 - 15% by weight.

Preferably, the collagen suspension is mixed with any component, preferably a drug, another biopolymer, a hormone, organic and inorganic particles and nanoparticles.

An object of the invention is the use of a homogeneous collagen suspension obtained by the method described above, for the production of homogeneous collagen materials.

Preferably, the homogeneous collagen materials are collagen materials of the desired shape, size, morphology and sterility, preferably the homogeneous collagen materials being in the form of a film, a cell substrate layer, a tissue substrate layer, a porous shape, a 3D substrate, a 3D print-out, an injectable material, a dressing material, a sorbent, a bioseparation material, a membrane for filtration/biofiltration, selective separation, adsorption, a catalyst, a bioink and a carrier for various types of active substances including drugs or fertilizers.

An object of the invention is a method for obtaining collagen-based hydrogel materials, characterized in that it comprises the following steps:
**I.** a homogeneous collagen suspension obtained by the method described above is homogenized and then, with mixing, preferably on a vortex, the solution is neutralized,
**II**. the solution obtained in step **I.** is cast into a container of the required shape,
**III**. the solution is dried until a dry material is obtained.

Preferably, in step **I.** the mixture is neutralized until it reaches a pH in the range 5-9, preferably 6-7, most preferably 7, preferably by adding a solution of a base, preferably NaOH, more preferably 1 M NaOH.

Preferably, the collagen is admixed with glycerol in an amount of 0.1 - 15% by weight.

Preferably, after step **I.** and before step **II.,** the collagen suspension is mixed with any component, preferably a drug, another biopolymer, a hormone, organic and inorganic particles and nanoparticles.

Preferably the method for obtaining collagen-based hydrogel materials, comprises the following steps:
**I.** a homogeneous collagen suspension obtained by the method described in one of the claims 1-5 is homogenized and then, with mixing, preferably on a vortex, is neutralized, the mixture is neutralized until it reaches a pH in the range 5-9, preferably 6-7, most preferably 7, preferably by adding a solution of a base, preferably NaOH, more preferably 1 M NaOH.
**II**. a desired amount of the solution obtained in step **I.** is transferred to a container of a desired shape and size,
**III**. after pouring, the container is tightly covered and placed in a drying oven at a temperature of 36 to 40°C, for a time until gelation occurs, preferably for 7 days,
**IV.** the supernatant is collected from above the material and replaced with ultra-pure water, the entire process is preferably repeated 3 times, the hydrogel obtained in this way can be used directly for research or further dried and stored for at least 30 days in refrigerated conditions until use.
**V.** optionally further drying mentioned in point **IV.** is carried out by placing the open container in the drying oven, preferably at a temperature of 36 to 40 °C, preferably for 7 to 20 days

An object of the invention is a method for obtaining a collagen film and/or layer and/or substrate for culture, characterized in that it comprises the following steps:
**I.** a homogeneous collagen suspension obtained by the method described above is homogenized, then, with mixing, preferably on a vortex, the solution is neutralized until it reaches a pH of 5 - 9, preferably 6 - 7, most preferably 7, wherein the neutralization is performed preferably by adding an NaOH solution, more preferably 1 M NaOH,
**II**. a desired amount of the solution is transferred to a container of a desired shape and size,
**III**. after pouring, the container is tightly covered and placed in a drying oven at a temperature of 36 to 40°C, for a time until gelation occurs, preferably for 7 days,
**IV.** after observing gelation, the container is opened and placed back in a laboratory drying oven at a temperature of 36 to 40°C for 7 days,
**V.** the film and/or layer and/or substrate for culture are further dried in a drying oven at a temperature of 36 to 40°C, preferably for 7 to 20 days.

Preferably, after step **V.,** a step of incubation in water is carried out to get rid of residues from synthesis.

Preferably, after step **V.,** a step of incubation is carried out, wherein the incubation is carried out in 70% ethylene alcohol, for additional cross-linking of the material and sterilization, then the resulting material is preferably rinsed in ultra-pure distilled water.

Preferably, after step **V.,** a step of incubation is carried out, wherein the incubation is carried out in a sterile culture medium for 24 hours.

An object of the invention is a method for obtaining collagen print-outs, characterized in that it comprises the following steps:
**I.** a homogeneous collagen suspension obtained by the method described above, without neutralization, is placed in a bioprinter cartridge, and then centrifuged to remove air bubbles, preferably at a speed of 700 - 2000 rpm, most preferably 1000 - 1500 rpm,
**II**. the printing process is carried out, preferably using an extrusion-based bioprinter,
**III**. the resulting print-outs are cross-linked by washing the ready print-outs with a solution composed of 1 M NaOH and DMSO, preferably mixed together in a ratio of 7:3, wherein the pH of the solution is in the range of 9 - 14, preferably 10 - 12, most preferably 11.

Preferably, to obtain print-outs with cells, the pH of the homogeneous suspension from step **I.** is adjusted to not less than 4 before mixing with the cells.

An object of the invention is a method for obtaining porous collagen shapes, characterized in that it comprises the following steps:
**I.** a homogeneous collagen suspension obtained by the method described above is homogenized, and then, while mixing, preferably on a vortex, the solution is neutralized, preferably to pH 7 using a solution of a base, preferably NaOH, more preferably using 1 M NaOH,
**II**. subsequently, a desired amount of the solution is poured into desired containers,
**III**. the material is preferably frozen at -80°C or in liquid nitrogen and then subjected to a lyophilization process to obtain porous shapes.

Preferably, after step **I.** and before step **II.,** the collagen suspension is mixed with any porogen known in the art, having the desired grain size and shape, the porogen preferably being a salt or a sugar.

An object of the invention is a method for obtaining porous collagen shapes, characterized in that it comprises the following steps:
**I.** a homogeneous collagen suspension obtained by the method described above is homogenized, then, with mixing, preferably on a vortex, the solution is neutralized until it reaches a pH of 5 - 9, preferably 6 - 7, most preferably 7, wherein the neutralization is preferably performed by adding an NaOH solution, more preferably 1 M NaOH, then mixed with a porogen having the desired grain size and shape, the porogen preferably being a salt or a sugar,
**II**. a desired amount of the solution is transferred to a container of a desired shape and size,
**III**. after pouring the liquid, the container is tightly covered and placed in a drying oven at a temperature of 36 to 40°C, for a time until gelation, preferably for 7 days,
**IV.** after observing gelation, the container is opened and placed back in a laboratory drying oven at a temperature of 36 to 40°C for 7 days,
**V.** the porous shapes are further dried in a drying oven at a temperature of 36 to 40°C, preferably for 7 to 20 days.

Preferably, after step **V.,** a step of incubation in water is carried out to get rid of residues from synthesis.

Preferably, after step **V.,** a step of incubation is carried out, wherein the incubation is carried out in 70% ethylene alcohol, for additional cross-linking of the material and sterilization, then the resulting material is preferably rinsed in ultra-pure distilled water.

Preferably, after step **V.,** a step of incubation is carried out, wherein the incubation is carried out in a sterile culture medium for 24 hours.

### Detailed description of proposed technical solution.

Collagen, derived from any source, preferably of type I, preferably derived from bovine tendons, is placed in a solution composed of a mild organic solvent and a buffer.

The concentration of collagen with respect to the solvent is 0.1 to 15% w/v. Preferably, to obtain a disordered matrix at the bottom of a culture well or Petri dish, the concentration is in the range of 0.1 to 5%; layers, films and membranes are obtained from a concentration range of 0.1 to 10%. 3D printing is possible with a concentration range of 5 - 15%.

The organic solvent should have a low surface tension with respect to collagen, preferably being dimethyl sulfoxide (DMSO), but any other solvent known in the art, for which the contact angle with respect to collagen is below 20°, is also acceptable.

The buffer used should contain salts naturally occurring in a living organism, preferably being a phosphate buffer (PBS), but other buffers known in the art are also acceptable.

The role of DMSO is optimal wetting of collagen; ions contained in the buffer are intended to partially disentangle the collagen structure and stabilize the fibrils. Both liquids can be mixed together in various proportions, preferably 5 parts DMSO to 1 part PBS, by volume.

Small amounts of acids are also added to the solution, preferably being HCl and HF, in such an amount that the final concentration of each of them in the solution is not higher than 0.1 M (as low as possible, but ensuring a homogeneous suspension, preferably 0.005 M of HCl, 0.012 M of HF). The minimum concentrations are 0.001 M of HCl and 0.001 M of HF.

The solution thus prepared is subsequently pre-mixed on a vortex, then placed on a magnetic stirrer, at a temperature of 2 - 8°C (preferably 4 - 6°C), for a period of 3 to 7 days (preferably 3).

After this time, the solution is acidified using an acid solution, preferably 1 M HCl, to a pH in the range of 2 to 4, preferably 2.5.

The acidified solution is mixed, preferably on a magnetic stirrer, most preferably on a vortex.

Delayed acidification with a low concentration of acid allows preserving the native structure of collagen - at the stage of adding acid, the collagen is already to some extent disentangled in the suspension, which minimizes the formation of local concentration gradients of acid ions that could denature its structure.

After lowering the pH, the solution is again vortexed and placed on a magnetic stirrer at a temperature of 2 - 8°C (preferably 4 - 6°C) for a time needed to further reduce the number of agglomerates in the solution. Preferably, the process of further mixing lasts 3 days. After this time, there are only small amounts of agglomerates in the suspension, difficult to detect with the naked eye.

In the next step, the suspension is subjected to homogenization - first mechanically, and then manually. For example, the following are used for this purpose: a Bio-Gen PRO200 Series mechanical tissue homogenizer equipped with a Multi-Gen 7XL tip and a Potter-Elvehjem-type manual Teflon homogenizer. Mechanical mixing is intended to break up larger structures, whereas manual homogenization leads to further homogenization and further breaking up of agglomerates.

The suspension thus prepared is ready for further processing, depending on the desired shape, using methods known in the art.

The suspensions processed into foils, films and membranes are subjected to a neutralization process using a solution of a base.

In an embodiment, neutralization may be performed by adding an NaOH solution until a pH of 5 - 9 (preferably 6-7, most preferably 7) is reached. Any other methods known in the art may also be used, including the use of another base or dialysis into a buffer, for example PBS.

Preferably, collagen may also be admixed with glycerol, in an amount of 0.1 - 15% by weight, which enhances flexibility of the resulting materials and thus their handling.

For the purpose of 3D printing, it is advantageous to carry out the neutralization process after imparting the suspension with the desired morphology. Rinsing of the printed shape may be performed using a solution composed of 1 M NaOH and DMSO mixed together in a ratio of 7:3. The pH of the solution should be in the range of 9 - 14, preferably 10 - 12, most preferably 11.

Previously known technical solutions provide much lower versatility and repeatability, and the techniques used do not fulfil the principles of green chemistry. Additionally, methods for preparing sterile materials are not discussed.

The materials provided in the present invention are easy to handle and easy to store.

The production techniques discussed in the examples allow for obtaining sterile materials on which the cell culturing can be carried out.

Compared to previously known technical solutions, the present application offers the following advantages:
1. use of solvents that are consistent with the idea of green chemistry
2. no need to use additional cross-linkers, the uncontrolled residues of which reduce biocompatibility of materials
3. no need to replace the solvent, or subsequent incubation of the already obtained material in another medium to prevent shrinkage
4. possibility of processing using many techniques, including 3D printing, ensuring greater versatility of the materials obtained and broadening the range of potential applications
5. greater cost-effectiveness and common availability of the reagents and techniques used
6. possibility of processing to any shape, morphology and size, using all methods known in the art - the solutions are homogeneous and their parameters are repeatable. Furthermore, it is possible to use different concentrations, advantageous for specific processing methods.

The technical solutions according to the invention differ from the technical solutions known in the art, among others, in the following:
1. The use of a precisely selected composition of the suspension, in which the solvents employed fit the idea of "green chemistry" and allow for obtaining a homogeneous collagen suspension, which then allows for obtaining cytocompatible, easy to handle, homogeneous materials.
2. Introduction of a stepwise change in pH. In the first phase, the collagen undergoes optimal wetting, mixing and coarse homogenization. Subsequently, after 3 - 7 days (preferably 3), lowering the pH to 2.5 and continuing mixing allows for obtaining optimally disentangled fibrils and a highly homogeneous suspension. Too rapid a reduction in pH would result in partial denaturation of the material due to the non-homogeneous interaction of collagen with hydrogen ions. On the other hand, failure to reduce pH after 3 - 7 days results in the collagen fibrils not being sufficiently disentangled. During gelation, the interactions between collagen molecules are thus inhomogeneous, which results in the formation of locations with locally increased and decreased collagen concentration. Materials obtained from such suspensions are discontinuous and mechanically unstable.
3. Mechanical homogenization at a later step of the method allows for the removal of any larger agglomerates. Its application at such a delayed stage of the method is not obvious and allows for obtaining homogeneous suspensions with optimally disentangled fibrils. Mechanical homogenization of the solution at earlier stages would result in significant, uncontrolled changes in the concentration of the solution, and thus would reduce the consistency of materials' preparation.
4. Obtaining mechanically stable print-outs from a bioprinter by rinsing already printed materials with a DMSO solution of NaOH having a pH in the range of 10 - 12. While neutralizing the pH to obtain gelled, water-stable films and layers is known in the art, the use of this process for 3D printing is neither known nor obvious. Suspending NaOH in DMSO is non-obvious and unpractised in the art, and is intended to reduce the rate of solvent exchange between the collagen solution and the fluid surrounding it, to precisely preserve the obtained shape. Washing with an aqueous solution of NaOH caused rapid diffusion, which resulted in cracking of the print-outs and their detachment from the substrate.

The technical solutions according to the invention provide, among others, the following advantages.
1. It is possible to obtain materials of any shape, size and morphology (films, matrices, layers, 3D structures).
2. Little harmful and mild solvents are used, in accordance with the idea of green chemistry.
3. It is possible to preserve the native conformation of collagen, and after processing the material is not soluble in water.
4. The need to use toxic cross-linkers is eliminated.
5. The resulting collagen materials of various shapes are cytocompatible, support cell adhesion - they are suitable for cell culturing.
6. The resulting materials are hydrogel materials, have a fibrous morphology, enabling cell migration into the interior of the material.
7. The resulting materials have a high surface development and are very absorbent, whereby they can be used as a sorbent, a carrier of the active substance.

### Examples of practical implementation of technical solution

Hydrogel collagen materials can be processed into a film, a layer, a cell substrate, porous shapes or 3D prints.

Advantageously, collagen can also be admixed with glycerol, in an amount of 0.1 - 15% by weight, which enhances the flexibility of the obtained materials, and thus their ease of handling. In all cases, before the forming step the collagen suspension can be mixed with any component to obtain a blend. Possible additives include inter alia: drugs, other biopolymers, hormones, organic and inorganic particles and nanoparticles. In this way, the material gains additional functions, for example becoming: a drug carrier, a catalyst, a sorbent, a bioactive material.
**i.** A specified amount of collagen should be weighed on a laboratory scale, placed in a container together with a magnetic stir bar. The collagen should then be covered with a PBS/DMSO solution in a volume ratio of 1:5, with an addition of HCl and HF. To obtain foils and films, depending on the desired thickness, the concentration of collagen in the solution can be in the range of 0.1 - 15% by weight relative to the volume of the solvent. Disordered substrates are obtained using the concentration range of 0.1 to 5%. Bioprinting bioink is preferably obtained at a concentration of 7% (but can be obtained using the range of 5 - 10%).
   Example suspension: 1 g collagen + 16 ml DMSO + 3.2 ml PBS + 100 µl 5% HF + 100 µl 1 M HCl. The mixture is pre-homogenized on a vortex.
**ii.** The solution thus prepared is placed on a magnetic stirrer, in a refrigerator (temperature 4°C), for 3 days.
**iii.** After 3 days, it is acidified to pH 2.5 using 1 M HCl. The acid is added gradually, with mixing on a vortex. Step **iii** is crucial to the manufacturing process, because it allows obtaining uniform and homogeneous suspensions in a highly repeatable manner.
**iv.** After acidification, the solution is again placed on a magnetic stirrer, in a refrigerator (temperature 4°C), for 3 days. After 3 days, a homogeneous suspension should be obtained, creamy-yellow in colour, with a small amount of agglomerates visible with the naked eye.
**v.** After 3 days, the suspension is removed from the refrigerator and homogenized with a mechanical homogenizer (approximately 1 minute). After homogenization, there should be no agglomerates visible in the solution. The solution prepared in this way can be stored in a refrigerator and should be used within 72 hours.

Just before use, the solution should be mixed on a vortex and homogenized again using a hand homogenizer.

When processing into thin layers, films, membranes, shapes and the like, the solution should be neutralized to a neutral pH, preferably 7. The method is most preferably practiced using 1 M NaOH solution. The base is added gradually with vortex mixing. This step is crucial because it initiates the gelation process.

Then, it is possible to add the desired additive, either by directly introducing the dry substance into the suspension or by adding a dispersion of this substance in DMSO. After adding the additive, the solution should be mixed again on a vortex.

The material takes the shape and form of the container in which it is cast.

### Example 1 - casting of films, layers, substrates for culture

**For casting films/layers/substrates for cell culturing.** The solution can be used to obtain standalone, easy to handle films that can be fit by cutting to any shape and size. The layers are obtained by casting a small amount of material onto hydrophilic surfaces. The volume of the cast suspension depends on the desired thickness of the final product and the dimensions of the hydrophilic surface. Besides that, it is possible to obtain sterile cell substrates directly in the wells of multi-well cell culturing plates. In the latter case, step vi. and casting should be performed under sterile conditions (in a laminar flow cabinet), and the drying of the film should take place in a previously sterilized drying oven.
**i.** After homogenization, the solution should be neutralized to pH 7 by addition of 1 M NaOH, with mixing on a vortex.
**ii.** Then, an appropriate amount of the solution should be pipetted into containers of the desired shape and size.
**iii.** After casting, the containers should be tightly covered and placed in a laboratory drying oven at 36 - 40°C for 7 days.
**iv.** After 7 days, the material will undergo gelation, which is manifested by a color change to off white. After observing gelation, the container with collagen should be opened and placed again in the laboratory drying oven at 36 - 40°C for 7 days (at low concentrations longer incubation of the material under cover may be necessary; containers should not be opened before observing initial gelation).
   In the case of preparing porous shapes, the material should be frozen at -80°C and subjected to the lyophilization process.
**v.** Further drying of the films and cell substrates takes place in a laboratory drying oven at 36 - 40°C, and usually takes 10 - 20 days (this depends on the volume of liquid being cast and the surface dimensions - which translate into the thickness of the final product). The material should not be removed from the drying oven before it dries (it should be dry to the touch).
**vi.** The material which is dry to the touch is suitable for further use. In the case of films, they can be removed from the containers in which they were cast and, properly secured, can be stored in a refrigerator for at least 6 months.
**vii.** The layers can be used for any purpose. In contact with water, the material becomes a hydrogel and increases its volume.
**viii.** All the materials contain residual salts, thus, before use an incubation step in water can be carried out to remove them from the material.
**ix.** An additional incubation in 70% ethyl alcohol can also be carried out. This treatment allows for additional cross-linking of the collagen structure and also sterilizes the material (after this step, rinsing in ultra-pure distilled water is recommended to remove residual alcohol).
**x.** Cell substrates can subsequently be used directly for a cell culturing. Before seeding the cells, the material should be incubated in a sterile culture medium in an incubator for 24 hours to adsorb the morphotic components of the medium. After this time, the hydrogel will hydrate. The medium in which it was incubated should be pipetted off, after which it is possible to seed the cells.

Steps **viii., ix.,** and **x.** can be carried out both together and separately, depending on need and purpose.

### Example 2 - obtaining porous shapes

**i.** There are two ways of preparing porous shapes - by lyophilization (a process known in the art) or by using a porogen (a process known in the art). It is also possible to combine these two processes. Porous shapes can be obtained using collagen concentrations in the range of 2 - 15% by weight.
**ii.** In the case of lyophilization, the homogenized polymer solution neutralized to pH 7 should be transferred to a container of the desired shape, frozen at -80°C (or in liquid nitrogen) and subjected to the lyophilization process.
**iii.** In the case of porogen use, the porogen (e.g., a salt, a sugar) of the desired grain size and shape should be mixed with collagen neutralized to pH 7 in the desired ratio. The mixture should then be transferred to a mould of the desired shape and subjected to steps **iii. - x.** from example 1.
**iv.** In the case of combining freeze-drying and the use of a solid-state porogen (which may constitute ice crystals, as in US5869080), the mixture from point **iii.** of this example should be frozen, freeze-dried, and then the salts should be washed out (point **viii.** of example 1).

### Example 3 - bioprinting ink

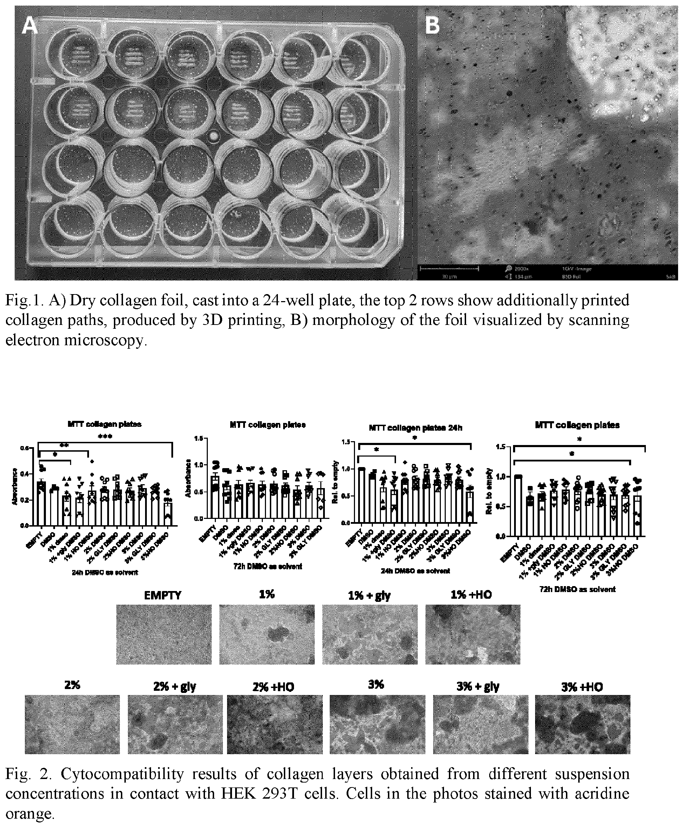

**i.** Collagen solutions at a concentration of 5 - 10% by weight are used for bioprinting.
**ii.** The solution without neutralization should be transferred to bioprinter cartridges, then centrifuged to remove air bubbles, preferably at a speed of 700 - 2000 rpm, most preferably 1000 - 1500 rpm. A properly processed ink should have rheological parameters similar to commercially available inks, that is, have rheological characteristics in which the phenomenon of shear thinning occurs, meaning a decrease in viscosity with an increase in shearing rate (Fig. 1). The second desirable phenomenon is a rapid return to high viscosity after the shearing process has ceased (Fig. 2). For example, _ and _ represent a comparison of 7% collagen suspension with the START ink from CELLINK, which has rheological properties optimized for 3D printing.
**iii.** The solution thus prepared can be used for up to 7 days, for printing using a bioprinter based on the extrusion technique, however, it is recommended to homogenize the suspension again, if it is not used within 24 hours.
**iv.** It is possible to mix the ink with cells and print directly. In this case, before mixing with cells, the mixture should be adjusted to a pH value not less than 4.
**v.** The obtained bioprint-outs are cross-linked by washing the ready print-outs with DMSO, to which a solution of NaOH in water with a pH between 10 and 12, preferably 11, has been added. The shapes can be repeatedly dried and rehydrated, used in the cell culturing.
**vi.** To modify mechanical properties, it is also possible to use additional chemical cross-linkers, including photo-cross-linkers.

### Example 4 - injectable collagen

**i.** Solutions at a collagen concentration of up to 10%, preferably 5%, are suitable for injection.
**ii.** Immediately before injection, the solution should be neutralized and transferred to a syringe.
**iii.** At this stage, it is possible to add a bioactive substance.
**vi.** Additional cross-linking of the suspension is possible.

### Example 5. - General description of the method of production and composition (for approximately 20 ml of suspension)

I. The collagen in powder form (in this example, type I collagen derived from bovine tendons, sample substrate: C9879 from Sigma Aldrich) is weighed on a laboratory scale and transferred to a plastic, screw-cap container with a flat bottom (for example, this could be a polypropylene container for bodily fluids, with a capacity of 60ml).
II. The powder is then mixed with 16ml of DMSO (in this example, DMSO with a purity of >99.0% from Avantor Chemicals) and 3.2ml of PBS (in this example, ROTI^{®}Cell PBS from Carl Roth). Subsequently, 100 µl 5% (by weight) HF + 100 µl 1 M HCl is added.
III. The mixture is initially homogenized using a vortex mixer - the container is sealed and placed on the vortex mixer for about one minute at a speed of approximately 2000 RPM.
IV. The prepared solution is then placed on a magnetic stirrer in a refrigerator (temperature 4 °C) for 6 days.
V. After 6 days, the mixture is acidified to a pH of 2.5 using 1M HCl, with mixing on a vortex mixer. The container is sealed and placed on the vortex mixer for about one minute at a speed of approximately 2000 RPM.
VI. After acidification, the solution is placed again on a magnetic stirrer in a refrigerator (temperature 4 °C ) for 3 days. After 3 days, a homogeneous suspension should be obtained, with a creamy-yellow colour and a small amount of agglomerates visible to naked eye.
   a. After 3 days, the suspension is removed from the refrigerator and homogenized using a mechanical stirrer (for approximately 1 minute). The mechanical stirrer used: Omni TH with Omni Tip 'Soft' attachment. After homogenization, no agglomerates should be visible in the solution. The prepared solution can be stored in the refrigerator and should be used by 72 hours.
VII. Subsequently, all possible additives are added to the suspension.

In the application developed by the Inventors, these are all or selected components from the list below:
a. Glycerol (for example, G9012 from Sigma Aldrich), typically used in an amount of 5% by weight relative to the weight of collagen.
b. Multi-walled, oxidized carbon nanotubes (CNTs) in a DMSO suspension. The CNTs used are described in a previous publication: https://doi.org/10.1039/D0NR09057C. Concentration of CNTs in DMSO: 7.5 mg/ml. The CNT suspension in DMSO is homogenized using an ultrasonic stirrer for 2 minutes (in our case, this is Vibra Cell, VCX 130 from Sonics & Materials, Inc., set to 30% power).
c. The target content of carbon nanotubes in the ink is 0.25% by weight relative to the weight of collagen.
d. Drugs, active substances or hormones. In the discussed application, these are preferably: dexamethasone (Dex), triiodothyronine (T3), palmitic acid conjugated to bovine serum albumin (BSA, produced by T in the laboratory). In the discussed application, the concentration of additives is preferably: 0.35% Dex, 0.06% T3, 8.89% BSA relative to the weight of collagen. Additives are introduced into the collagen suspension after being dissolved in DMSO or ultra-pure water. Concentrations of prepared suspensions: Dex: 7.35 mg/ml, T3: 1.22 mg/ml, BSA: 133.33 mg/ml. It is advisable to select the highest possible concentrations of active substances, which allows avoiding excessive dilution of the solution.
e. Instead of adding directly to the collagen suspension, Dex and T3 can also be added to a previously prepared suspension of carbon nanotubes in DMSO. In this situation, a drug suspension in DMSO is prepared and mixed with the CNTs suspension. For the production of material containing both drugs, 90 µl of the CNTs suspension is mixed with 257 µl of the first drug suspension, and 90 µl of the CNTs suspension is mixed with 257 µl of the second drug suspension. Both solutions are then combined, homogenized, and added to the collagen suspension. To prepare material with a single drug, 180 µl of the CNTs suspension is mixed with 257 µl of the drug suspension.

VIII. Just before use, the solution should be mixed on a vortex mixer and re-homogenized using a hand homogenizer. The hand homogenizer used: glass, conical or straight, with or without a cap, from Lab-Szklo, with a capacity of 20 ml. The solution is placed in the homogenizer tube and ground with circular piston movements for about 5 minutes.

IX. The prepared suspension can then be transferred to cartridges for 3D printing or neutralized using a 1M solution of base, in our case NaOH, and cast into appropriate containers.

### Example 6 - Method of the realisation of the layers

1. Layers are typically obtained in the concentration range of 0.1 - 1% by weight of collagen relative to the weight of the entire solution. In a typical, non-limiting example, the suspension prepared according to the recipe described above has a concentration of 1% by weight. Sample composition for point I of Example 5: 0.2 g collagen + 16ml DMSO + 3.2 ml PBS + 100 µl 5% HF + 100 µl 1 M HCl.
2. After step VII of Example 5, the suspension is neutralized - under the control of a pH meter (in our case, Mettled Toledo S400), to a pH in the range of 6-8, using 1M NaOH solution.
3. Exemplary volume and a vessel for point IX of Example 5 - 150µl of collagen suspension is poured into a well of a 24-well plate.
4. After placing the liquid in the wells, the plate is covered with its lid and placed in a laboratory drying oven at a temperature of 40°C for 7 days. To ensure sterile materials, the drying oven should be preheated to 100°C for at least 12 hours.
5. After 7 days, the material will gel, which is manifested by a colour change from transparent to whitish. After observing gelation, the container with collagen should be opened and placed again in the laboratory drying oven at 40 °C for 7 days (at low concentrations, it may be necessary to incubate the material longer under cover, the containers should not be opened before observing initial gelation).
6. Further drying of the materials is carried out in a laboratory drying oven at a temperature of 40°C and typically lasts 7-14 days. The material should not be removed from the drying oven before it is dry (it should be dry to the touch).
7. Once dry to the touch, the material is ready for further use. The dry layers can be stored in 24-well plates, tightly sealed in a refrigerator. We have observed material stability for a minimum period of 6 months.
8. An example appearance of collagen layers with added glycerol, without other additives, cast in a 24-well plate, is shown in Fig. 1. An SEM image showing the fibrous morphology of the layers is also presented.
9. All materials contain residual salts; before use, a water incubation step can be performed to remove synthesis residues.
10. An exemplary incubation process is conducted as follows:
   1. Filling the culture plate wells with ultrapure water and leave for 5 minutes.
   2. Exchanging of the water to 70 ethanol solution, leave for 1 min
   3. Exchanging of the ethanol for culture medium (the one to be used in the cell culturing), incubation in a cell culturing incubator for 24h.
   4. Sucking the medium out, cell seeding.
11. To maintain sterile culture, points 1-10 are carried out in a sterile room and under a laminar chamber. Sub-points 3 and 4 of point 10 are only performed when using the foil for the cell culturing.
12. Examples of the results of the cytocompatibility of the layers against HEK 293T cells are shown in Fig. 2. The experiment concerned the optimization of the concentration of cast substrates, therefore it also contains concentrations higher than those presented in the patent application - however, they were rejected in the subsequent stages of research.

### Example 7 - Method of the realisation of the foil

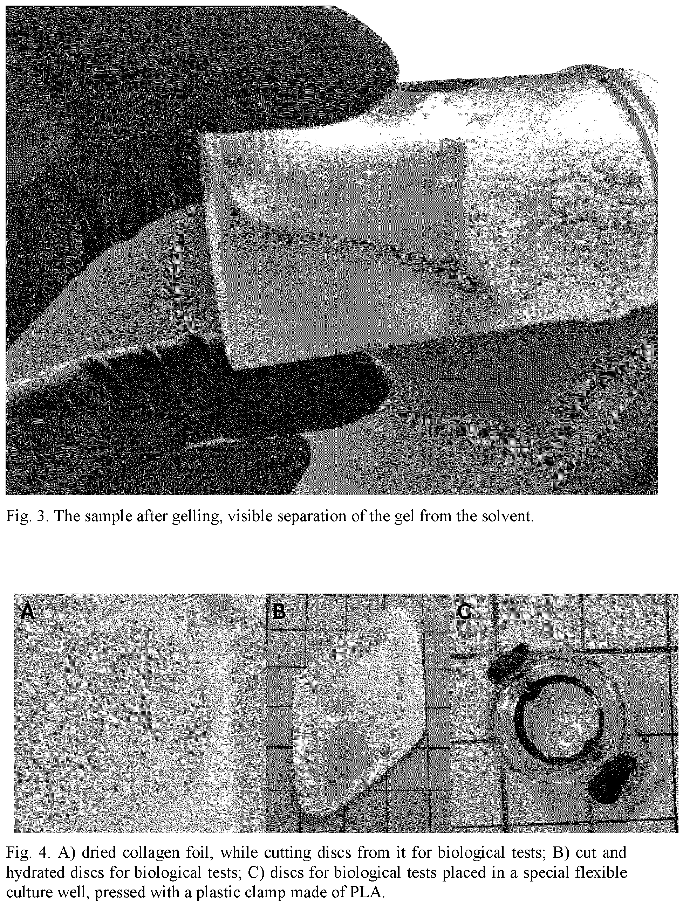

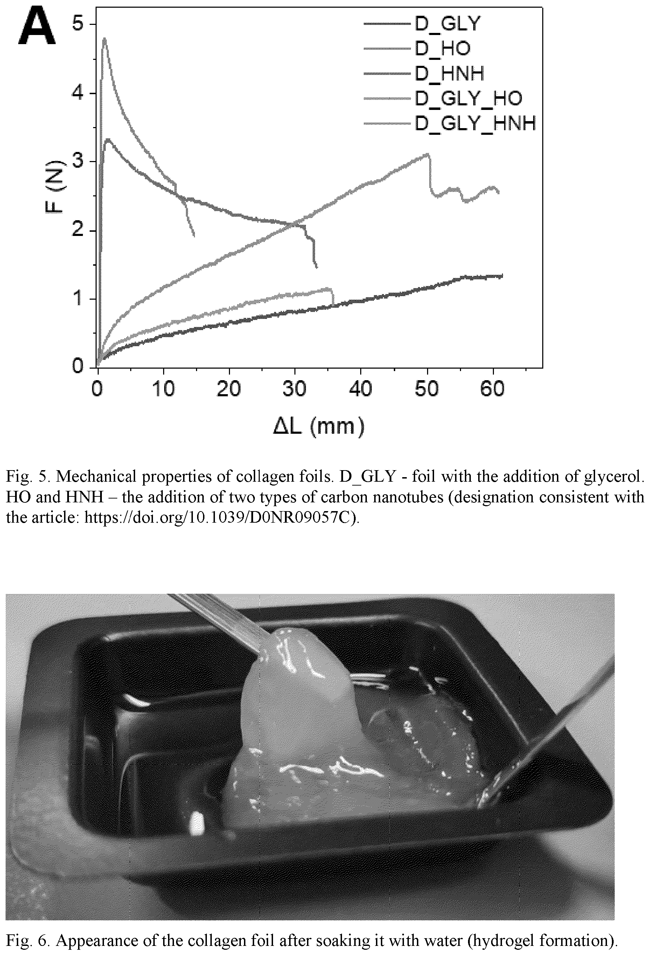

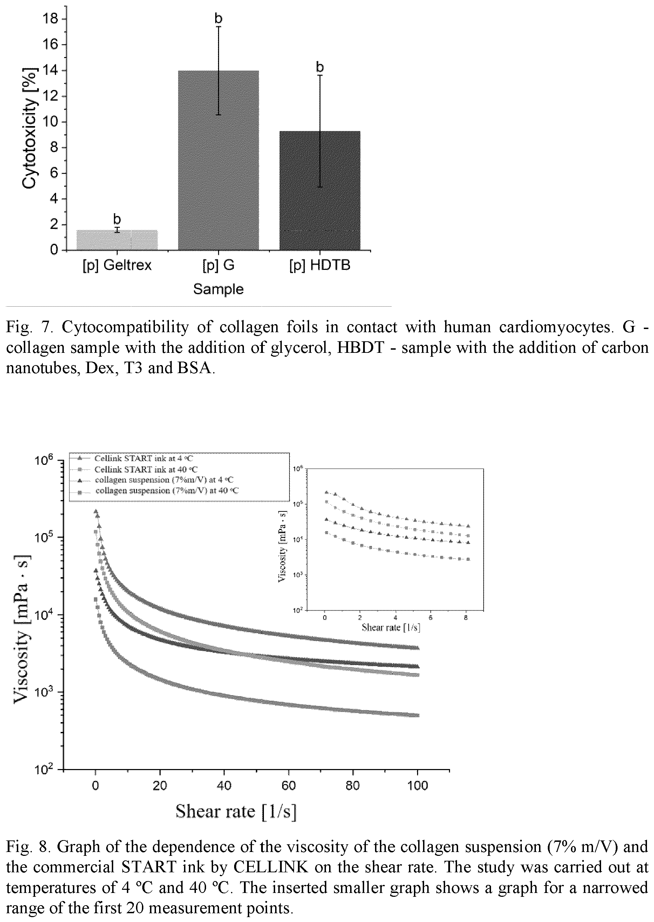

1. Films are typically obtained with a concentration range of 1 - 5% by weight of collagen relative to the weight of the entire solution. In our typical example, the suspension prepared according to the recipe described above has a concentration of 3% by weight. Example composition for point I of Example 5: 1 g of collagen + 16 ml DMSO + 3.2 ml PBS + 100 µl 5% HF + 100 µl 1 M HCl.
2. After step VII of Example 5, the suspension is neutralized - under the control of a pH meter (in our case, Mettled Toledo S400), to a pH in the range of 6-8, using 1M NaOH solution.
3. Exemplary volume and a vessel for point IX of Example 5 - 5.26 ml of collagen suspension is poured into a wide-bottomed weighing vessel made of polystyrene, with a volume of 50 ml. Examples of dishes we use: https://www.eaglelabdish.com/HexagonalWeighDishes.asp
4. After placing the liquid in the vessel, it is tightly covered and placed in a laboratory drying oven at 40 °C for 7 days.
5. After 7 days, the material will gel, which is indicated by a colour change from transparent to whitish (and the separation of the hydrogel from the liquid, as seen in Fig. 3). Upon observing the gelation, the collagen container should be opened and placed back in the laboratory drying oven at 40°C for 7 days (at low concentrations, longer incubation under cover may be necessary; the containers should not be opened before observing initial gelation).
6. Further drying of the materials is carried out in a laboratory drying oven at a temperature of 40°C and typically lasts 7-14 days. The material should not be removed from the drying oven before it is dry (it should be dry to the touch).
7. Once dry to the touch, the material is ready for further use and can be removed from the container and placed in a tightly sealed zip-lock bag. We have observed material stability for a minimum period of 6 months.
8. Samples of the desired shapes and sizes can then be cut from the film, as shown in Fig. 4.
9. In the dry state, the materials have mechanical handleability, allowing them to be manipulated without the risk of damage. The mechanical properties of the film are shown in Fig. 5.
10. All materials contain residual salts; before use, a water incubation step can be performed to remove synthesis residues.
11. Exemplary incubation process:
   1. Cutting discs to the desired shape and size (note that the hydrated hydrogel increases its dimensions by approximately 75% in all directions).
   2. Placing the discs in the desired vessel (for example, this could be a well of a culture plate).
   3. Filling the culture plate wells with ultrapure water and leaving for 5 minutes. After swelling, the sample becomes a handy hydrogel, as shown in Fig. 6.
   4. Exchanging of the water to 70 ethanol solution, leaving for 1 min.
   5. Exchanging of the ethanol for culture medium (the one to be used in the cell culturing), incubating in a cell culturing incubator for 24h
   6. Sucking the medium out, cell seeding.
12. To maintain a sterile culture, steps 2-6 of point 11 are conducted in a sterile room and under a laminar flow cabinet. Steps 5 and 6 are performed only when the film is used for the cell culturing. The cytocompatibility of materials in contact with human cardiomyocytes, obtained by differentiating induced stem cells, is shown in Fig. 7.

### Example 8 - Method of the realisation of the ink for 3 D printing

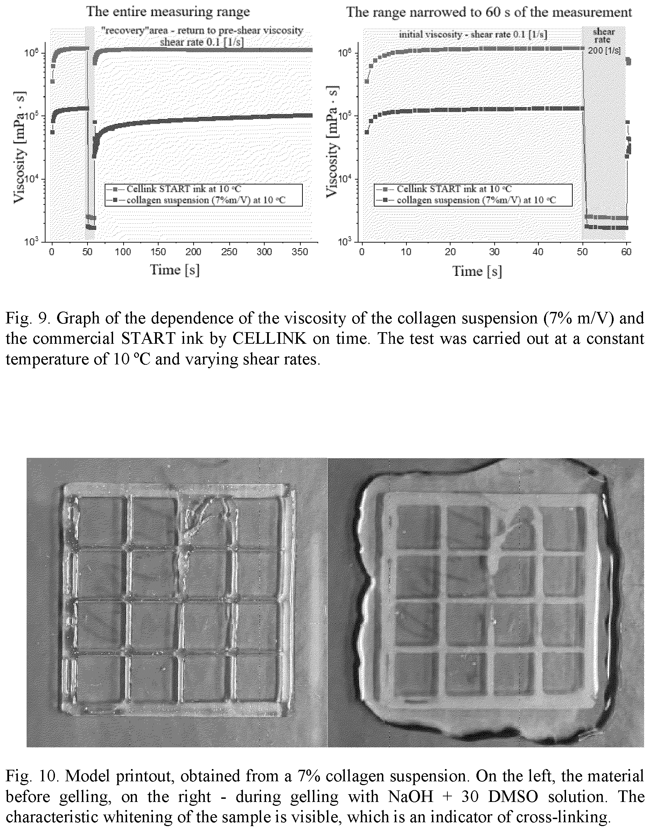

1. 3D printing inks are typically obtained with a concentration range of 5 - 10% by weight of collagen relative to the weight of the entire solution. In our typical example, the suspension prepared according to the recipe described above has a concentration of 3% by weight. Sample composition for point I of Example 5: 2.3 g collagen + 16ml DMSO + 3.2 ml PBS + 100 µl 5% HF + 100 µl 1 M HCl.
2. In this case, the suspension obtained in point VIII of Example 5 is not neutralized, but transferred directly to the cartridge (syringe) of a 3D printer using the extrusion technique (bioprinter, in our case Cellink BioX).
3. After transferring to a syringe, it is placed in a centrifuge and centrifuged for 5 minutes at a drum speed of 2000/min. This is aimed at removing of air bubbles that interrupt the continuity of the printing stream.
4. After centrifugation, the syringe is placed in the 3D printer and the process of printing forms of the desired shape and size is performed.
5. Optimized printing parameters:
   1. Printing temperature of the substrate: 7°C
   2. Suspension temperature (extruder): 10°C
   3. To obtain high printing resolution, the diameter of the printing nozzle is 25 Ga
   4. Printing pressure: 8kPa
6. The rheological properties of 7% ink, depending on temperature and compared to the commercially available Cellink START ink, are presented in Fig. 8 and Fig. 9.

The ink intended for 3D printing should have rheological properties that involve the phenomenon of shear thinning. This allows the material to be extruded from the nozzle. As can be seen in Fig. 8, the obtained collagen ink has a shear-rate dependence of viscosity analogous to commercially available inks - it is shear thinning as the viscosity decreases with increasing shear rate.

During the second measurement of rheological properties, the speed of the ink's return to its initial viscosity was tested after shear cessation. This feature allows the inks to maintain the given shape after printing - the faster the return to high viscosity, the less blurring of the print under the influence of gravity and its own weight. The measurement was carried out at a constant temperature of 10 °C, and the collagen ink was again compared to the commercially available Cellink START ink. After shearing ceases, both suspensions immediately regain their initial viscosity, which proves that they have rheological properties optimal for 3D printing.

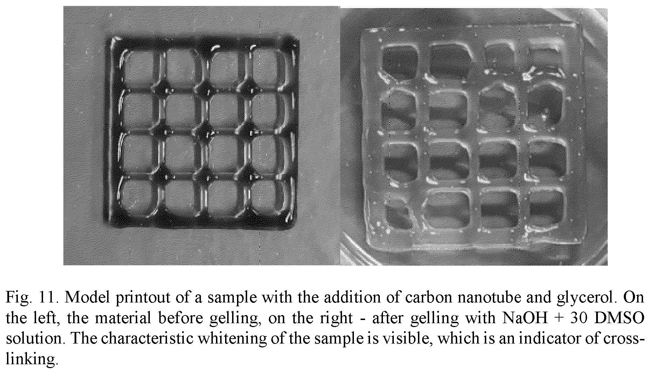

7. Collagen gelation occurs by immersing the print in a NaOH solution with a pH of 11, doped with a 30% addition of DMSO (which slows the diffusion process and solvent exchange between the print and the surrounding liquid). It is also possible to print directly into the neutralizing liquid. Fig. 10 shows an example print obtained from a 7% collagen suspension (with glycerol). Fig. 11 shows the same model obtained using a collagen suspension with the addition of carbon nanotubes (and glycerol).

## Claims

1. A method for preparing a homogeneous collagen suspension, **characterized in that** it comprises the following steps
**I.** to collagen, with mixing, preferably on a vortex, a solution composed of a mild organic solvent and a buffer, with the addition of acids is added wherein:
- as the collagen preferably type I collagen is used, preferably it is the collagen derived from bovine tendons,
- the concentration of the collagen with respect to the solvent is in the range of 0.1 to 15% w/v,
- the organic solvent is a solvent exhibiting low surface tension relative to the collagen, preferably the solvent is a solvent for which the contact angle value for collagen is below 20°, most preferably the organic solvent is dimethyl sulfoxide (DMSO),
- the buffer is a buffer containing salts naturally occurring in a living organism, preferably the buffer is a phosphate buffer (PBS),
- the solvent and the buffer can be mixed together in various proportions, preferably the solvent and the buffer are mixed in a ratio of 5 parts of the solvent to 1 part of the buffer, by volume, most preferably 5 parts DMSO to 1 part PBS, by volume,
- the acids are HCl and HF, wherein the concentration of each of the acid in the solution is not higher than 0.1 M,
**II**. subsequently, the solution obtained in step I. is mixed at a temperature of 2 - 8°C, preferably 4 - 6°C, for a period of 3 to 7 days, preferably for a period of 3 days,
**III**. subsequently, the solution obtained in step **II.,** while mixing, preferably on a vortex, is gradually acidified using an acid solution, to a pH in the range of 2 to 4, preferably to pH 2.5, preferably the acid solution is 1 M HCl,
**IV.** subsequently, the solution obtained in step **III.** is mixed at a temperature of 2 - 8°C, preferably 4 - 6°C, for a time necessary to further reduce the number of agglomerates in the solution, preferably, for a period of 3 days,
**V.** subsequently, the suspension obtained in step **IV.** is homogenized to obtain a homogeneous collagen suspension, preferably first the suspension being homogenized mechanically, and then the mixture being homogenized manually.

2. The method according to claim 1, **characterized in that** in step **I.** the concentration of the collagen with respect to the solvent is in the range from 0.1 to 10% w/v, preferably from 0.1 to 5% w/v, equally preferably in the range from 5 to 10% w/v, more preferably 7% w/v, or preferably from 5 to 15% w/v.

3. The method according to any one of claims 1 - 2, **characterized in that** in step **I.** HCl and HF acids are added, wherein the minimum concentration of HCl is 0.001 M and the minimum concentration of HF is 0.001 M, more preferably the concentration of HCl is 0.005 M and the concentration of HF is 0.012 M.

4. The method according to any one of claims 1-3, **characterized in that** in step **V.** the collagen is admixed with glycerol in an amount of 0.1 - 15% by weight.

5. The method according to any one of claims 1-3, **characterized in that**, in step **V.** the collagen suspension is mixed with any component, preferably a drug, another biopolymer, a hormone, organic and inorganic particles and nanoparticles.

6. Use of a homogeneous collagen suspension obtained by the method described in any one of claims 1-5, for the production of homogeneous collagen materials.

7. The use according to claim 6, **characterized in that** the homogeneous collagen materials are collagen materials of the desired shape, size, morphology and sterility, preferably the homogeneous collagen materials being in the form of a film, a cell substrate layer, a tissue substrate layer, a porous shape, a 3D substrate, a 3D print-out, an injectable material, a dressing material, a sorbent, a bioseparation material, a membrane for filtration/biofiltration, selective separation, adsorption, a catalyst, a bioink and a carrier for various types of active substances including drugs or fertilizers.

8. A method for obtaining collagen-based hydrogel materials, **characterized in that** it comprises the following steps:
**I.** a homogeneous collagen suspension obtained by the method according to any one of claims 1 - 5 is homogenized and then, with mixing, preferably on a vortex, is neutralized, preferably the mixture is neutralized until it reaches a pH in the range 5-9, preferably 6-7, most preferably 7, preferably by adding a solution of a base, preferably NaOH, more preferably 1 M NaOH,
**II**. the solution obtained in step **I.** is cast into a container of the required shape,
**III**. the solution is dried until a dry material is obtained,

9. The method for obtaining collagen-based hydrogel materials, **characterized in that** it comprises the following steps:
**I.** a homogeneous collagen suspension obtained by the method described in one of the claims 1-5 is homogenized and then, with mixing, preferably on a vortex, is neutralized, preferably the mixture is neutralized until it reaches a pH in the range 5-9, preferably 6-7, most preferably 7, preferably by adding a solution of a base, preferably NaOH, more preferably 1 M NaOH.
**II**. a desired amount of the solution obtained in step **I.** is transferred to a container of a desired shape and size,
**III**. after pouring, the container is tightly covered and placed in a drying oven at a temperature of 36 to 40°C, for a time until gelation occurs, preferably for 7 days,
**IV.** the supernatant is collected from above the material and replaced with ultra-pure water, the entire process is preferably repeated 3 times, the hydrogel obtained in this way can be used directly for research or further dried and stored for at least 30 days in refrigerated conditions until use,
**V.** optionally further drying mentioned in point **IV.** is carried out by placing the open container in the drying oven, preferably at a temperature of 36 to 40 °C, preferably for 7 to 20 days.

10. A method for obtaining a collagen film and/or layer and/or substrate for culture, **characterized in that** it comprises the following steps:
**I.** the homogeneous collagen suspension obtained by the method according to any one of claims 1 - 5 is homogenized, and then, with mixing, preferably on a vortex, is neutralized, preferably the mixture is neutralized until it reaches a pH in the range 5 - 9, , preferably 6 - 7, most preferably 7, preferably by adding a solution of a base, preferably NaOH, more preferably 1 M NaOH,
**II**. a desired amount of the solution is transferred to a container of a desired shape and size,
**III**. after pouring, the container is tightly covered and placed in a drying oven at a temperature of 36 to 40°C, for a time until gelation, preferably for 7 days,
**IV.** after observing gelation, the container is opened and placed back in a laboratory drying oven at a temperature of 36 to 40°C for 7 days,
**V.** further drying is carried out by placing the open container in the drying oven, preferably at a temperature of 36 to 40 °C, preferably for 7 to 20 days.

11. A method for obtaining porous collagen shapes, **characterized in that** it comprises the following steps:
**I.** a homogeneous collagen suspension obtained by the method according to any one of claims 1 - 5 is homogenized, then, with mixing, preferably on a vortex, the solution is neutralized, until it reaches a pH in a range of 5 - 9, preferably 6-7, most preferably 7, wherein the neutralization is performed preferably by adding an NaOH solution, more preferably 1 M NaOH,
**II**. then the suspension can be mixed with any porogen known in the art having the desired grain size and shape, the porogen preferably being a salt or a sugar
**III**. a desired amount of the solution is transferred to a container of a desired shape and size,
**IV.** after pouring, the container is tightly covered and placed in a drying oven at a temperature of 36 to 40°C, for a time until gelation, preferably for 7 days,
**V.** the supernatant is collected from above the material and replaced with ultra-pure water, the entire process is repeated at least 3 times, until observing the washing out of all of the potentially used porogen grains, in the case of porogen used in the form of water-soluble grains,
**VI.** the porous shapes are further dried in a drying oven at a temperature of 36 to 40°C, preferably for 7 to 20 days, or the material is frozen preferably at -80°C or in liquid nitrogen and then subjected to a lyophilization process to obtain porous shapes.

12. A method for obtaining collagen print-outs, **characterized in that** it comprises the following steps:
**I.** a homogeneous collagen suspension obtained by the method according to any one of claims 1-5, without neutralization, is placed in a bioprinter cartridge, and then centrifuged to remove air bubbles, preferably at a speed of 700 - 2000 rpm, most preferably 1000 - 1500 rpm,
**II**. the printing process is carried out, preferably using an extrusion-based bioprinter,
**III**. the resulting print-outs are cross-linked by washing the ready print-outs with a solution composed of 1 M NaOH and DMSO, preferably mixed together in a ratio of 7:3, wherein the pH of the solution is in the range of 9 - 14, preferably 10 - 12, most preferably 11.

13. The method according to claims 9 - 11, **characterized in that**, after step **V.,** a step of incubation is carried out, wherein the incubation is carried out:
in water to get rid of residues from synthesis and/or
in 70% ethylene alcohol, for additional cross-linking of the material and sterilization, then the resulting material is preferably rinsed in ultra-pure distilled water and/or in a sterile culture medium for 24 hours.

14. The method according to claim 13, **characterized in that** in order to obtain print-outs with cells, the pH of the homogeneous suspension from step **I.** is adjusted to not less than 4 before mixing with the cells.
